# EUROPEAN PATENT APPLICATION

(11) **EP 4 793 270 A1**
(43) Date of publication of application: **19.08.2026**
(21) Application number: 24876678.4
(22) Date of filing: 12.10.2024
(51) Int. Cl.: C07D 211/88, C07C 227/12, C07C 229/08, C07C 227/18

(54) **PREPARATION METHOD FOR PREGABALIN INTERMEDIATE**

(30) Priority: 13.10.2023 CN 202311324317
(71) Applicant: Zhejiang Huahai Pharmaceutical Co., Ltd., Taizhou, Zhejiang 317024 (CN)
(72) Inventor: WANG, Yougui, Linhai Taizhou, Zhejiang 317024 (CN); CHEN, Hui, Linhai Taizhou, Zhejiang 317024 (CN); CAI, Zhangqing, Linhai Taizhou, Zhejiang 317024 (CN); HUANG, Liping, Linhai Taizhou, Zhejiang 317024 (CN); ZHU, Yuanxun, Linhai Taizhou, Zhejiang 317024 (CN); HUANG, Wenfeng, Linhai Taizhou, Zhejiang 317024 (CN); ZHU, Wei, Linhai Taizhou, Zhejiang 317024 (CN)
(74) Representative: ZBM Patents - Zea, Barlocci & Markvardsen
(86) International application number: PCT/CN2024/124402
(87) International publication number: WO 2025/077873

(57) **Abstract**

The present invention relates to a synthesis method for a pregabalin intermediate, which effectively improves the reaction yield and the product purity, significantly reduces the impurity content and is suitable for large-scale industrial production.

## Description

### TECHNICAL FIELD

The present application relates to the field of organic synthesis technology, and in particular to a method for synthesizing a Pregabalin intermediate.

### BACKGROUND

Pregabalin is a novel gamma-aminobutyric acid (GABA) receptor agonist used for treating neuropathic pain, focal onset seizures and anxiety disorder. 3-Isobutylglutarimide (compound V) is an important intermediate in the preparation of Pregabalin, and a common preparation route is as follows:

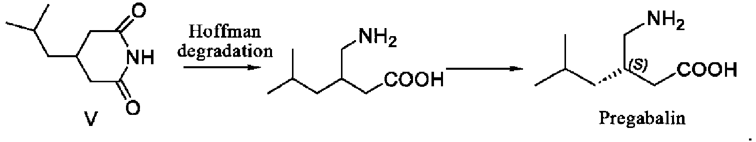

Patent CN100410242C discloses a method for preparing compound V: carrying out a cyclization reaction of 3-isobutylglutaric acid and a nitrogen-containing reagent under a solvent-free condition at 100 °C to 200 °C, and recrystallizing using a mixed solution of alcohol and water. However, in an actual large-scale industrial production, the purity and yield of compound V obtained by this method are relatively low.

### SUMMARY

One aspect of the present application provides a method for preparing compound V, wherein the method comprises the following step:
step 3: reacting compound IV with a nitrogen-containing reagent in the presence of an acid a to obtain compound V,

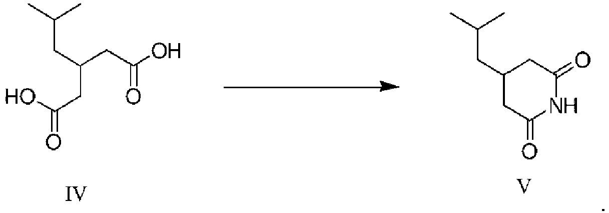

In some embodiments of the present application, the nitrogen-containing reagent in step 3 is one or more of urea, ammonium bicarbonate and ammonium chloride;
the acid a in step 3 is one or more of organic acids or inorganic acids, preferably one or more of phosphoric acid, hydrochloric acid, sulfuric acid, nitric acid, oxalic acid, methanesulfonic acid, p-toluenesulfonic acid, formic acid, acetic acid and trifluoroacetic acid, more preferably one or more of phosphoric acid, methanesulfonic acid, p-toluenesulfonic acid, hydrochloric acid, acetic acid and trifluoroacetic acid;
an amount of the nitrogen-containing reagent in step 3 is 0.5 to 1.5 molar equivalents of compound IV; and
an amount of the acid a in step 3 is 0.02 to 0.5 molar equivalents of compound IV.

In some embodiments of the present application, a reaction in step 3 is carried out in an organic solvent A.

In some embodiments of the present application, the organic solvent A in step 3 is one or more of toluene, xylene, 1,2-dimethoxyethane and ethyl benzoate; preferably, the organic solvent A is toluene or xylene, more preferably, the organic solvent A is toluene; a concentration of compound IV in the organic solvent A is 1.2 to 4 mol/L.

A temperature of the reaction in step 3 is 105 °C to 145 °C; preferably, the temperature of the reaction in step 3 is 110 °C to 130 °C (e.g., 110 °C, 115 °C, 120 °C, 125 °C, 130 °C, or any number or range therein); and
a time of the reaction in step 3 is 10 h to 20 h, preferably 13 h to 18 h.

In some embodiments of the present application, the method for preparing compound V further comprises the following step 4:
step 4: after completion of the reaction, cooling for crystallization, filtering, and then adding a solvent for slurrying, and filtering to obtain compound V.

In some embodiments of the present application, the solvent in step 4 is an organic solvent B or a mixture of the organic solvent B and water; preferably, the solvent in step 4 is acetone; and
a temperature of the slurrying is -5 °C to 50 °C; preferably, the temperature of the slurrying is 20 °C to 50 °C.

In some embodiments of the present application, the method for preparing compound V further comprises the following step:
step 2: reacting compound III in the presence of an acid b to obtain compound IV,

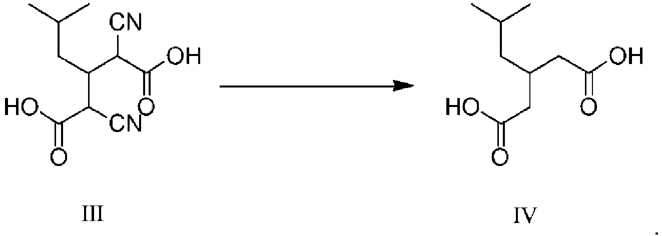

In some embodiments of the present application, the acid b in step 2 is one or more of hydrochloric acid, sulfuric acid and trifluoroacetic acid;
a temperature of the reaction in step 2 is 90 °C to 130 °C;
a time of the reaction in step 2 is 8 h to 30 h; preferably, the time of the reaction in step 2 is 15 h to 25 h; and
a solvent for the reaction in step 2 is water.

In some embodiments of the present application, the method for preparing compound V further comprises the following step:
step 1: reacting compound I with compound II in an alkali solution to obtain compound III,

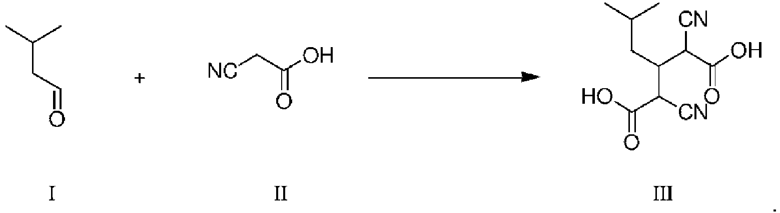

In some embodiments of the present application, the alkali in step 1 is one or two of an organic base and an inorganic base; preferably, the organic base in step 1 is one or more of triethylamine, diethylamine, n-propylamine, diisopropylethylamine, isopropylamine, morpholine, N-methylmorpholine, piperidine, pyridine and piperazine; preferably, the inorganic base in step 1 is an alkali metal hydroxide, such as sodium hydroxide, potassium hydroxide; and

a temperature of the reaction in step 1 is 0 °C to 10 °C.

In some embodiments of the present application, after completion of the reaction in step 1, without post-treatment, the reaction in step 2 is carried out directly.

In some embodiments of the present application, after completion of the reaction in step 2, the organic solvent A is added to extract the reaction solution, an organic phase is separated, 40% to 60% (e.g., 40%, 50%, 60%, or any number or range therein) of the organic solvent A is removed by distillation, and the next step reaction is continued.

The organic solvent A removed by distillation can be recovered and reused.

Another aspect of the present application provides a method for preparing Pregabalin, wherein the method comprises the following step:
reacting compound IV with the nitrogen-containing reagent in the presence of the acid a to obtain compound V 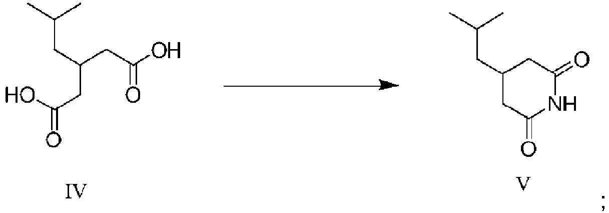
and further preparing Pregabalin from compound V.

In the present application, under acid catalysis, the cyclization reaction of compound IV is carried out in the organic solvent A (e.g., toluene, xylene, 1,2-dimethoxyethane, and ethyl benzoate). The reaction can be effectively carried out at 110 °C, representing a mild condition, and it does not require a high temperature of 150 °C to 180 °C. This avoids the accumulation of energy, temperature, or pressure caused by solvent-free reactions at the high temperatures, and avoids localized overheating of the system leading to material charring and an increase in the content and types of reaction impurities. The method of the present application not only improves the reaction yield but also facilitates safe production. The solvent can be recovered and reused after distillation, making it more economical and environmentally friendly.

After the completion of the reactions in steps 1 to 2, only simple treatment is required, and the next step reaction can be carried out without isolation and purification. The reactions in steps 1 to 3 can be carried out in one reactor, simplifying the production process. After the completion of the reaction in step 3, it is only necessary to cool to precipitate a solid, and then purify a crude product by slurrying to obtain a high-purity product. The total yield of the three-step reaction can reach up to 88.9%. The overall process route is simple to operate and advantageous for industrial production.

The preparation method provided by the present application effectively improves the reaction yield and product purity, significantly reduces the impurity content, and is suitable for large-scale industrial production.

### DETAILED DESCRIPTION

Terms in the present application, unless otherwise specified, have the following meanings:
The terms "organic solvent A" and "organic solvent B" in the present application essentially still refer to the organic solvents, wherein A and B are used only to distinguish the organic solvents applied in different reactions therein.

The terms "acid a" and "acid b" in the present application essentially still refer to acids, wherein a and b are used only to distinguish the acids applied in different reactions therein.

In the present application, proton nuclear magnetic resonance (¹H-NMR) was measured using a Bruker nuclear magnetic resonance spectrometer.

In the present application, unless otherwise specified, "% concentration" refers to mass percentage concentration.

In the present application, "concentrated hydrochloric acid" refers to an aqueous hydrochloric acid solution with a concentration of 36% to 38%.

In the present application, "yield relative to compound IV" refers to the yield of step 3 alone.

The impurity contents in the examples of the present application were detected using high performance liquid chromatography (HPLC), and the HPLC detection conditions are as follows:
chromatographic column: XBridge Premier BEH C18 150*4.6mm
mobile phase A: 1.0 mL phosphoric acid to 1000 mL water
mobile phase B: 1.0 mL phosphoric acid to 1000 mL acetonitrile
wavelength: 210 nm
flow rate: 0.8 mL/min.

In the present application, "RRT" is relative retention time, which generally refers to the ratio of the retention time of the component to be tested relative to the retention time of the main component.

To make the technical problems, technical solutions and beneficial effects solved by the present application clearer, the present application is further described below with reference to specific examples. However, it should be understood that the examples are not intended to limit the scope of the present application.

The reagents and solvents of the present application were purchased from commercial sources. Unless otherwise specified in the present application, all reagents and solvents were used directly without treatment.

### Example 1

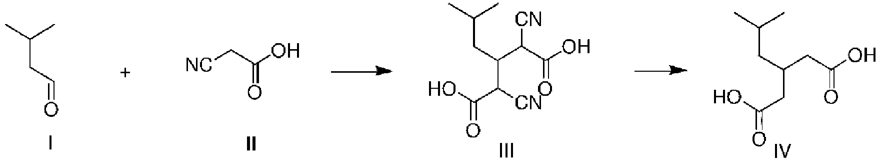

Step 1: 58.0 g of solid sodium hydroxide was dissolved in 255 mL of water, and the solution was cooled to 3 °C. 142.5 g of 70% cyanoacetic acid was slowly added dropwise while maintaining the temperature at 5 °C. Then, 50.0 g of isovaleraldehyde was added dropwise to the solution, and the reaction was maintained for 1 h.

Step 2: 353 g of concentrated hydrochloric acid was added to the reaction system, the temperature was heated to 110 °C, and the reaction was maintained for 24 h. The reaction solution was extracted with 800 mL of toluene, and was allowed to stand for layer separation. Then, the aqueous phase was removed, the organic phase was retained, and about 400 mL of toluene was removed by distillation to obtain a toluene solution of compound IV. The content of compound IV therein was measured by high performance liquid chromatography (HPLC).

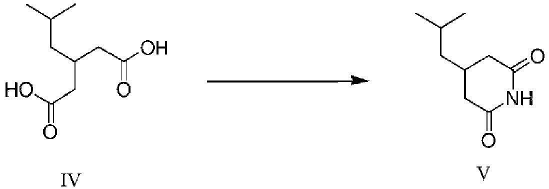

Step 3: 35 g of urea and 8 g of p-toluenesulfonic acid were added to the above toluene solution, the temperature was heated to 110 °C, and the reaction was refluxed for 18 h. After completion of the reaction, the mixture was cooled to 5 °C to precipitate a solid, and then filtered. Then, 15 mL of acetone and 150 mL of pure water were added. The mixture was slurried at 40 °C, cooled to room temperature, filtered and dried to obtain 85.3 g of compound V with a purity of 99.8% and a yield relative to compound IV of 96%. The total yield of the three-step reaction was 86.9%.

### Characterization of compound IV

¹H-NMR (CDCl₃): δ 8.37 (s, 1H), 2.69-2.24 (dd, 4H), 2.22 (m, 1H), 1.27 (m, 1H), 1.26 (t, 2H), 0.91-0.90 (s, 6H).

### Example 2

Step 1: 220 g of solid sodium hydroxide was dissolved in 1 L of water, and the solution was cooled to 5 °C. 500 g of 70% cyanoacetic acid was slowly added dropwise to the solution while maintaining the temperature at 5 °C. Then, 145 g of isovaleraldehyde was added dropwise to the solution, and the reaction was maintained for 1 h.

Step 2: 920 g of concentrated sulfuric acid was added to the reaction system, the temperature was heated to 110 °C, and the reaction was maintained for 20 h. The reaction solution was extracted with 2 L of xylene, and was allowed to stand for layer separation. Then, the aqueous phase was removed, the organic phase was retained, and about 1 L of xylene was removed by distillation to obtain a xylene solution of compound IV. The content of compound IV therein was measured by HPLC.

Step 3: 320 g of urea and 20 g of the concentrated hydrochloric acid were added to the above xylene solution, the temperature was heated to 130 °C, and the reaction was refluxed for 20 h. After completion of the reaction, the mixture was cooled to 10 °C to precipitate a solid, and then filtered. Then, 180 mL of acetone and 1500 mL of pure water were added. The mixture was slurried at 30 °C, cooled to room temperature, filtered and dried to obtain 250.1 g of compound V with a purity of 99.8% and a yield relative to compound IV of 96.5%. The total yield of the three-step reaction was 87.8%.

### Example 3

Step 1: 240 g of solid potassium carbonate was dissolved in 400 mL of water, and the solution was cooled to 7°C. 145 g of 70% cyanoacetic acid was slowly added dropwise to the solution while maintaining the temperature at 7 °C. Then, 55 g of isovaleraldehyde was added dropwise to the solution, and the reaction was maintained for 3 h.

Step 2: 420 g of concentrated hydrochloric acid was added to the reaction system, the temperature was heated to 110 °C, and the reaction was maintained for 17 h. The reaction solution was extracted with 1.4 L of xylene, and was allowed to stand for layer separation. Then, the aqueous phase was removed, the organic phase was retained, and about 0.7 L of xylene was removed by distillation to obtain a xylene solution of compound IV. The content of compound IV therein was measured by HPLC.

Step 3: 76 g of urea and 18 g of acetic acid were added to the above xylene solution, the temperature was heated to 130 °C, and the reaction was refluxed for 15 h. After completion of the reaction, the mixture was cooled to 10 °C to precipitate a solid, and then filtered. Then, 150 mL of acetone and 1.2 L of pure water were added. The mixture was slurried at 35 °C, cooled to room temperature, filtered and dried to obtain 93.6 g of compound V with a purity of 99.8% and a yield relative to compound IV of 95.8%. The total yield of the three-step reaction was 86.7%.

### Example 4

Step 1: 180 g of morpholine was diluted with 200 mL of water, and the solution was cooled to 5 °C. 100 g of cyanoacetic acid was slowly added dropwise to the solution while maintaining the temperature at 5 °C. Then, 60 g of isovaleraldehyde was added dropwise to the solution, and the reaction was maintained for 5 h.

Step 2: 320 g of trifluoroacetic acid was added to the reaction system, the temperature was heated to 110 °C, and the reaction was maintained for 16 h. The reaction solution was extracted with 1.2 L of ethyl benzoate, and was allowed to stand for layer separation. Then, the aqueous phase was removed, the organic phase was retained, and about 0.6 L of ethyl benzoate was removed by distillation to obtain an ethyl benzoate solution of compound IV. The content of compound IV therein was measured by HPLC.

Step 3: 85 g of urea and 42 g of methanesulfonic acid were added to the above ethyl benzoate solution, the temperature was heated to 130 °C, and the reaction was refluxed for 10 h. After completion of the reaction, the mixture was cooled to 0 °C to precipitate a solid, and then filtered. Then, 200 mL of acetone and 1.9 L of pure water were added. The mixture was slurried at 25 °C, cooled to room temperature, filtered and dried to obtain 102.7 g of compound V with a purity of 99.7% and a yield relative to compound IV of 96.6%. The total yield of the three-step reaction was 87.1%.

### Example 5

Step 1: 65 kg of solid sodium hydroxide was dissolved in 240 L of water, and the solution was cooled to 5 °C. 100 kg of 70% cyanoacetic acid was slowly added dropwise to the solution while maintaining the temperature at 5 °C. Then, 52 kg of isovaleraldehyde was added dropwise to the solution, and the reaction was maintained for 1.5 h.

Step 2: 390 kg of concentrated hydrochloric acid was added to the reaction system, the temperature was heated to 115 °C, and the reaction was maintained for 16 h. The reaction solution was extracted with 1100 L of xylene, and was allowed to stand for layer separation. Then, the aqueous phase was removed, the organic phase was retained, and about 550 L of xylene was removed by distillation to obtain a xylene solution of compound IV. The content of compound IV therein was measured by HPLC.

Step 3: 38 kg of urea and 8 kg of phosphoric acid were added to the above xylene solution, the temperature was heated to 130 °C, and the reaction was refluxed for 13 h. After completion of the reaction, the mixture was cooled to 8 °C to precipitate a solid, and then filtered. Then, 100 L of acetone and 1300 L of pure water were added. The mixture was slurried at 25 °C, cooled to room temperature, filtered and dried to obtain 90.3 kg of compound V with a purity of 99.8% and a yield relative to compound IV of 97%. The total yield of the three-step reaction was 88.3%.

### Example 6

Step 1: 200 g of solid sodium hydroxide was dissolved in 1 L of water, and the solution was cooled to 5 °C. 500 g of 70% cyanoacetic acid was slowly added dropwise to the solution while maintaining the temperature at 5 °C. Then, 145 g of isovaleraldehyde was added dropwise to the solution, and the reaction was maintained for 1 h.

Step 2: 1030 g of concentrated sulfuric acid was added to the reaction system, the temperature was heated to 110 °C, and the reaction was maintained for 20 h. The reaction solution was extracted with 2 L of toluene, and was allowed to stand for layer separation. Then, the aqueous phase was removed, the organic phase was retained, and about 1 L of toluene was removed by distillation to obtain a toluene solution of compound IV. The content of compound IV therein was measured by HPLC.

Step 3: 360 g of ammonium bicarbonate and 40 g of p-toluenesulfonic acid were added to the above toluene solution, the temperature was heated to 120 °C, and the reaction was refluxed for 16 h. After completion of the reaction, the mixture was cooled to 10 °C to precipitate a solid, and then filtered. Then, 180 mL of acetone and 1500 mL of pure water were added. The mixture was slurried at 20 °C, cooled to room temperature, filtered and dried to obtain 248.6 g of compound V with a purity of 99.8% and a yield relative to compound IV of 95.9%. The total yield of the three-step reaction was 87.3%.

### Example 7

Step 1: 200 g of solid sodium hydroxide was dissolved in 1 L of water, and the solution was cooled to 5 °C. 500 g of 70% cyanoacetic acid was slowly added dropwise to the solution while maintaining the temperature at 5 °C. Then, 145 g of isovaleraldehyde was added dropwise to the solution, and after the addition was completed, the reaction was maintained for 1 h.

Step 2: 920 g of concentrated sulfuric acid was added to the reaction system, the temperature was heated to 110 °C, and the reaction was maintained for 20 h. The reaction solution was extracted with 2 L of xylene, and was allowed to stand for layer separation. Then, the aqueous phase was removed, the organic phase was retained, and about 1 L of xylene was removed by distillation to obtain a xylene solution of compound IV. The content of compound IV therein was measured by HPLC.

Step 3: 270 g of ammonium chloride and 35 g of acetic acid were added to the above xylene solution, the temperature was heated to 130 °C, and the reaction was refluxed for 15 h. After completion of the reaction, the mixture was cooled to 5 °C to precipitate a solid, and then filtered. Then, 180 mL of acetone and 1500 mL of pure water were added. The mixture was slurried at 20 °C, cooled to room temperature, filtered and dried to obtain 250.6 g of compound V with a purity of 99.7% and a yield relative to compound IV of 96.7%. The total yield of the three-step reaction was 88.0%.

### Example 8

Step 1: 110 kg of solid sodium hydroxide was dissolved in 500 L of water, and the solution was cooled to 5 °C. 250 kg of 70% cyanoacetic acid was slowly added dropwise to the solution while maintaining the temperature at 5 °C. Then, 73 kg of isovaleraldehyde was added dropwise, and the reaction was maintained for 1 h.

Step 2: 460 kg of concentrated sulfuric acid was added to the reaction system, the temperature was heated to 110 °C, and the reaction was maintained for 20 h. The reaction solution was extracted with 1000 L of xylene, and was allowed to stand for layer separation. Then, the aqueous phase was removed, the organic phase was retained, and about 500 L of xylene was removed by distillation to obtain a xylene solution of compound IV. The content of compound IV therein was measured by HPLC.

Step 3: 160 kg of urea and 11 kg of concentrated hydrochloric acid were added to the above xylene solution, the temperature was heated to 130 °C, and the reaction was refluxed for 20 h. After completion of the reaction, the mixture was cooled to 10 °C to precipitate a solid, and then filtered. Then, 90 L of acetone and 750 L of pure water were added. The mixture was slurried at 25 °C, cooled to room temperature, filtered and dried to obtain 126.1 kg of compound V with a purity of 99.7% and a yield relative to compound IV of 97.3%. The total yield of the three-step reaction was 88.5%.

### Example 9

Step 1: 170 kg of morpholine was diluted with 200 L of water, and the solution was cooled to 5 °C. 100 kg of 70% cyanoacetic acid was slowly added dropwise to the solution while maintaining the temperature at 5 °C. Then, 60 kg of isovaleraldehyde was added dropwise, and the reaction was maintained for 5 h.

Step 2: 320 kg of trifluoroacetic acid was added to the reaction system, the temperature was heated to 110 °C, and the reaction was maintained for 15 h. The reaction solution was extracted with 1200 L of toluene, and was allowed to stand for layer separation. Then, the aqueous phase was removed, the organic phase was retained, and about 600 L of toluene was removed by distillation to obtain a toluene solution of compound IV. The content of compound IV therein was measured by HPLC.

Step 3: 85 kg of urea and 42 kg of methanesulfonic acid were added to the above toluene solution, the temperature was heated to 130 °C, and the reaction was refluxed for 10 h. After completion of the reaction, the mixture was cooled to 0 °C to precipitate a solid, and then filtered. Then, 200 L of acetone and 1900 L of pure water were added. The mixture was slurried at 20 °C, cooled to room temperature, filtered and dried to obtain 103.5 kg of compound V with a purity of 99.8% and a yield relative to compound IV of 97.4%. The total yield of the three-step reaction was 87.8%.

### Example 10

Step 1: 1100 kg of solid sodium hydroxide was dissolved in 5000 L of water, and the solution was cooled to 0 °C. 2500 kg of 70% cyanoacetic acid was slowly added dropwise to the solution while maintaining the temperature at 10 °C. Then, 730 kg of isovaleraldehyde was added dropwise, and the reaction was maintained for 2 h.

Step 2: 4600 kg of concentrated sulfuric acid was added to the reaction system, the temperature was heated to 110 °C, and the reaction was maintained for 24 h. The reaction solution was extracted with 8000 L of toluene, and was allowed to stand for layer separation. Then, the aqueous phase was removed, the organic phase was retained, and about 3000 L of toluene was removed by distillation to obtain a toluene solution of compound IV. The content of compound IV therein was measured by HPLC.

Step 3: 700 kg of urea and 80 kg of trifluoroacetic acid were added to the above toluene solution, the temperature was heated to 115 °C, and the reaction was refluxed for 15 h. After completion of the reaction, the mixture was cooled to 10 °C to precipitate a solid, and then filtered. Then, 900 L of acetone and 6000 L of pure water were added. The mixture was slurried at 20 °C for 2 hours, filtered and dried to obtain 1275.0 kg of compound V with a purity of 99.8% and a yield relative to compound IV of 97.7%. The total yield of the three-step reaction was 88.9%.

### Comparative Example 1

Step 1: 65 kg of solid sodium hydroxide was dissolved in 240 L of water, and the solution was cooled to 5 °C. 100 kg of 70% cyanoacetic acid was slowly added dropwise to the solution while maintaining the temperature at 5 °C, Then, 52 kg of isovaleraldehyde was added dropwise, and the reaction was maintained for 1.5 h.

Step 2: 390 kg of concentrated hydrochloric acid was added to the reaction system, the temperature was heated to 115 °C, and the reaction was maintained for 16 h. The reaction solution was extracted with 1100 L of xylene, and was allowed to stand for layer separation. Then, the aqueous phase was removed, the organic phase was retained, and about 550 L of xylene was removed by distillation to obtain a xylene solution of compound IV. The content of compound IV therein was measured by HPLC.

Step 3: 38 kg of urea was added to the above xylene solution, the temperature was heated to 130°C, and the reaction was refluxed for 13 h. After completion of the reaction, the mixture was cooled to 8°C to precipitate a solid, and then filtered. Then, 100 L of acetone and 1300 L of pure water were added. The mixture was slurried at 25°C, cooled to room temperature, filtered and dried to obtain 79.9 kg of compound V with a purity of 95.8% and a yield relative to compound IV of 86.1%. The total yield of the three-step reaction was 78.2%.

### Comparative Example 2

Step 1: 110 kg of solid sodium hydroxide was dissolved in 500 L of water, and the solution was cooled to 5 °C. 250 kg of 70% cyanoacetic acid was slowly added dropwise to the solution while maintaining the temperature at 5 °C. Then, 73 kg of isovaleraldehyde was added dropwise, and the reaction was maintained for 1 h.

Step 2: 460 kg of concentrated sulfuric acid was added to the reaction system, the temperature was heated to 110°C, and the reaction was maintained for 20 h. The reaction solution was extracted with 1000 L of xylene, and was allowed to stand for layer separation. Then, the aqueous phase was removed, the organic phase was retained, and about 500 L of xylene was removed by distillation to obtain a xylene solution of compound IV. The content of compound IV therein was measured by HPLC.

Step 3: 160 kg of urea was added to the above xylene solution, the temperature was heated to 130°C, and the reaction was refluxed for 20 h. After completion of the reaction, the mixture was cooled to 10°C to precipitate a solid, and then filtered. Then 90 L of acetone and 750 L of pure water were added. The mixture was slurried at 25°C, cooled to room temperature, filtered and dried to obtain 113.4 kg of compound V with a purity of 94.9% and a yield relative to compound IV of 87.4%. The total yield of the three-step reaction was 79.5%.

### Comparative Example 3

Step 1: 170 kg of morpholine was diluted with 200 L of water, and the solution was cooled to 5°C. 100 kg of cyanoacetic acid was slowly added dropwise to the solution while maintaining the temperature at 5°C. Then, 60 kg of isovaleraldehyde was added dropwise, and the reaction was maintained for 5 h.

Step 2: 320 kg of trifluoroacetic acid was added to the reaction system, the temperature was heated to 110°C, and the reaction was maintained for 15 h. The reaction solution was extracted with 1200 L of toluene, and was allowed to stand for layer separation. Then, the aqueous phase was removed, the organic phase was retained, and about 600 L of toluene was removed by distillation to obtain a toluene solution of compound IV. The content of compound IV therein was measured by HPLC.

Step 3: 85 kg of urea was added to the above toluene solution, the temperature was heated to 130°C, and the reaction was refluxed for 10 h. After completion of the reaction, the mixture was cooled to 0°C to precipitate a solid, and then filtered. Then 200 L of acetone and 1900 L of pure water were added. The mixture was slurried at 20°C, cooled to room temperature, filtered and dried to obtain 90.9 kg of compound V with a purity of 95.7% and a yield relative to compound IV of 85.5%. The total yield of the three-step reaction was 77.1%.

### Comparative Example 4

Compound V was prepared according to the method of CN100410242C: 200.0 g (1.06 mol) of 3-isobutylglutaric acid and 63.8 g (1.06 mol) of urea were added to a 1000 mL three-necked flask. The mixture was heated in an oil bath to 160 °C, reacted at 160 °C to 180 °C for 2 hours, then cooled to 90 °C. 400 mL of water and 400 mL of ethanol were directly added under stirring, and 15 g of activated carbon may be added. Then the mixture was heated to reflux for 30 minutes and filtered while hot. The filtrate was cooled, the solution was filtered off, and the crystals were dried under vacuum at 50 °C to obtain 152.3 g of compound V with a yield of 78.3% and a purity of 92.4%.

The specific impurity contents in the final products of the Examples and Comparative Examples are shown in the following table:

| | Unknown Impurity 1(RRT 0.272) | Compound IV (RRT 0.758) |
|---|---|---|
| Example 1 | 0.04% | 0 |
| Example 2 | 0.04% | 0 |
| Example 3 | 0.04% | 0 |
| Example 4 | 0.04% | 0 |
| Example 5 | 0.04% | 0 |
| Example 6 | 0.04% | 0 |
| Example 7 | 0.04% | 0 |
| Example 8 | 0.04% | 0 |
| Example 9 | 0.04% | 0 |
| Comparative Example 1 | 3.26% | 0.66% |
| Comparative Example 2 | 4.01% | 0.52% |
| Comparative Example 3 | 3.53% | 0.59% |
| Comparative Example 4 | 0.42% | 2.5% |

## Claims

1. A method for preparing compound V, wherein the method comprises the following step:
step 3: reacting compound IV with a nitrogen-containing reagent in the presence of an acid a to obtain compound V,

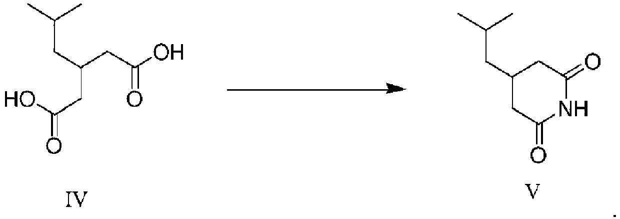

2. The method according to claim 1, wherein,
the nitrogen-containing reagent in step 3 is one or more of urea, ammonium bicarbonate and ammonium chloride;
the acid a in step 3 is one or more of an organic acid or an inorganic acid, preferably one or more of phosphoric acid, hydrochloric acid, sulfuric acid, nitric acid, oxalic acid, methanesulfonic acid, p-toluenesulfonic acid, formic acid, acetic acid and trifluoroacetic acid, more preferably one or more of phosphoric acid, methanesulfonic acid, p-toluenesulfonic acid, hydrochloric acid, acetic acid and trifluoroacetic acid;
an amount of the nitrogen-containing reagent in step 3 is 0.5 to 1.5 molar equivalents of compound IV; and
an amount of the acid a in step 3 is 0.02 to 0.5 molar equivalents of compound IV.

3. The method according to claim 1, wherein a reaction in step 3 is carried out in an organic solvent A.

4. The method according to claim 3, wherein the organic solvent A in step 3 is one or more of toluene, xylene, 1,2-dimethoxyethane and ethyl benzoate; preferably, the organic solvent A is toluene or xylene; more preferably, the organic solvent A is toluene;
a concentration of compound IV in the organic solvent A in step 3 is 1.2 to 4 mol/L;
a temperature of the reaction in step 3 is 105 °C to 145 °C, preferably 110 °C to 130°C; and
a time of the reaction in step 3 is 10 h to 20 h, preferably 13 h to 18 h.

5. The method according to claim 1, wherein the method for preparing compound V further comprises the following step 4:
step 4: after completion of the reaction, cooling for crystallization, filtering, and then adding a solvent for slurrying, and filtering to obtain compound V.

6. The method according to claim 5, wherein,
the solvent in step 4 is an organic solvent B or a mixture of the organic solvent B and water, preferably, the solvent in step 4 is acetone; and
a temperature of the slurrying in step 4 is -5 °C to 50 °C, preferably 20 °C to 50°C.

7. The method according to any one of claims 1 to 6, wherein the method for preparing compound V further comprises the following step:
step 2: reacting compound III in the presence of an acid b to obtain compound IV,

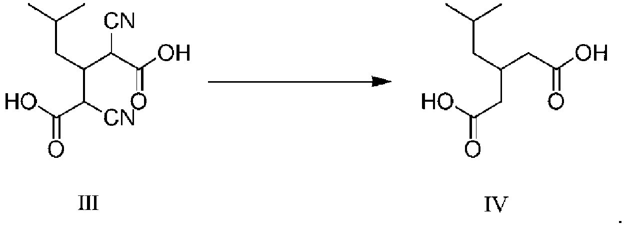

8. The method according to claim 7, wherein:
the acid b in step 2 is one or more of hydrochloric acid, sulfuric acid and trifluoroacetic acid;
a temperature of the reaction in step 2 is 90 °C to 130 °C;
a time of the reaction in step 2 is 8 h to 30 h, preferably 15 h to 25 h; and
a solvent for the reaction in step 2 is water.

9. The method according to claim 7, wherein the method for preparing compound V further comprises the following step:
step 1: reacting compound I with compound II in an alkali solution to obtain compound III,

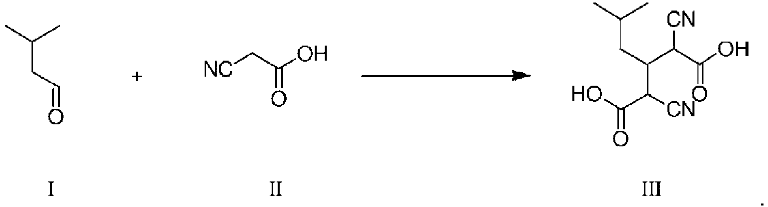

10. The method according to claim 9, wherein:
the alkali in step 1 is one or two of an organic base and an inorganic base, preferably one or more of triethylamine, diethylamine, n-propylamine, diisopropylethylamine, isopropylamine, morpholine, N-methylmorpholine, piperidine, pyridine and piperazine; more preferably an alkali metal hydroxide; and
a temperature of the reaction in step 1 is 0 °C to 10 °C.

11. A method for preparing Pregabalin, wherein the method comprises the following step:
step 3: reacting compound IV with a nitrogen-containing reagent in the presence of an acid a to obtain compound V, 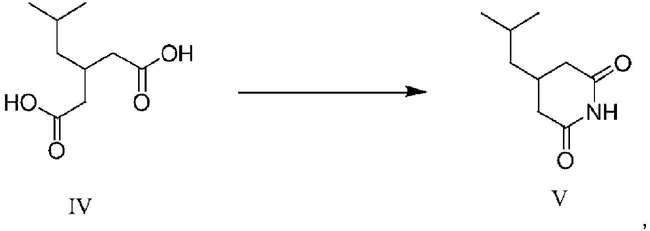
and further preparing Pregabalin from compound V.
